Europäisches Patentamt

**European Patent Office**

Office européen des brevets

(11) Publication number: **0 326 946**
**A2**

(12) # EUROPEAN PATENT APPLICATION

(21) Application number: **89101352.6**

(22) Date of filing: **26.01.89**

(51) Int. Cl.⁴: **C07C 7/00 , C07C 11/167 , C07C 11/06 , C07C 11/18**

(30) Priority: **01.02.88 US 150816**

(43) Date of publication of application:
**09.08.89 Bulletin 89/32**

(84) Designated Contracting States:
**BE DE GB**

(71) Applicant: **AIR PRODUCTS AND CHEMICALS, INC.**

**Allentown, PA 18195(US)**

(72) Inventor: **Andre, Robert Stevens**
**Box 209, RD No. 4**
**Coopersburg, PA 18036(US)**

(74) Representative: **Dipl.-Ing. Schwabe, Dr. Dr. Sandmair, Dr. Marx**
**Stuntzstrasse 16**
**D-8000 München 80(DE)**

(54) **Method and apparatus for olefinic separation.**

(57) A process and apparatus for separating selected $C_3$-$C_5$ olefinic products from combinations thereof with lower boiling point compounds comprises pressurizing a vapor stream, cooling the pressurized stream to produce condensate, flash evaporating the condensate and distilling the liquid fraction of the flash evaporated condensate, all at selected temperatures and pressures to produce a liquid stream in which is recovered a high percentage of the desired olefinic product available in the feed stream.

EP 0 326 946 A2

## METHOD AND APPARATUS FOR OLEFINIC SEPARATION

### Technical Field

The present invention pertains to the separation of $C_3$-$C_5$ olefinic product from lower boiling point compounds in an effective and efficient manner.

### Background of the Invention

Olefinic products such as propylene, butylene, isobutylene, butadiene, pentane, pentadiene, isopentadiene (referred to generally herein as $C_3$-$C_5$ olefins or olefinic products) are commonly produced in catalytic dehydrogenation processes using propane, butane, pentane or mixed paraffins as starting materials. Such processes include the CATOFIN and CATADIENE processes developed commercially by Air Products and Chemicals, Inc. of Allentown, Pa., the assignee of the present application Product streams from these dehydrogenation processes usually also include lighter or higher boiling point gases than the desired olefinic product.

Typically, the olefinic product has been separated from the lighter gases in such streams, in the past, by a product separation sequence which included pressurization of the olefinic-rich stream, absorption of the lighter gases from that stream (in an absorption medium, such as heavy oil) and then the stripping of the lighter gases from the absorption medium by distillation. Because absorption took place in this process in the vapor phase, entrained liquid was typically removed during and after compression (from the compression train product and in some cases from intermediate compression stages) by simply permitting such entrained liquid to collect and condense as the vapors passed through a drum or small tank, sometimes referred to a as a "flash drum" or "knockout drum." This combination of absorber/stripper and distillation column for stabilizing (or "deethanizing" or "depropanizing" as it was sometimes referred to) was effective to recover a high proportion of the available olefinic product in the feed stream However, its cost effectiveness was greatly impaired by the required size and energy demands of the absorber/stripper.

Accordingly, it has also been proposed prior to the present invention to substitute a cryogenic or cooling sequence for the absorber/stripper and thus to produce a cooled condensate from the compressed olefinicrich feed stream free of at least some of the light gases initially combined therewith This condensate, together with entrained liquid from the compression train would be separated in a distillation column, as in prior separation processes, into a liquid stream rich in the desired olefinic product and a vapor stream comprised primarily of lighter gases.

It has now been determined that the economics of the cryogenic process can be improved considerably in that the condensate produced by cooling includes a relatively high proportion of lower boiling compounds (as compared to the desired olefinic product). This greatly impairs the efficiency of product recovery by distillation.

### Brief Description of the Invention

Briefly, in the process and apparatus of the present invention, $C_3$-$C_5$ olefinic product is efficiently separated from lighter gases by compressing the vapor phase mixture (typically in a multi-stage compressor train) of the desired $C_3$-$C_5$ olefinic product and lighter gases to about 100-500 psig. The pressurized output of the compressor train is then cooled to form a liquid condensate stream with only residual light ends (1 to 30% by weight). Entrained liquid removed from the gaseous feed during or just after compression bypasses the cooling stage and is combined with condensate downstream of the cooling stage. This total liquid condensate stream is flash evaporated to (at 0-200 psig) remove remaining light ends (gases having a lower boiling point than the desired product) combined with the $C_3$-$C_5$ olefinic product. The remaining condensate from the flash evaporation step is then distilled at a pressure at 50-450 psig depending on the desired olefinic product. Generally, the vapor product of the flash evaporation stage is recycled to the compression stage, in some cases in combination with the distillation stage overhead stream.

Preferably, gas entering the cryogenic or cooling stage is compressed to about 100-200 psig (for C4 recovery processes) and cooled sufficiently to form a liquid/condensate stream comprised of 1 to 30% of gases having a boiling point lower than that of the desired product.

The cooled and condensed stream, at 50-150°F is flash evaporated adiabatically at 0-200 psig (preferably at 50-150 psig) and distilled at about the same or higher pressure.

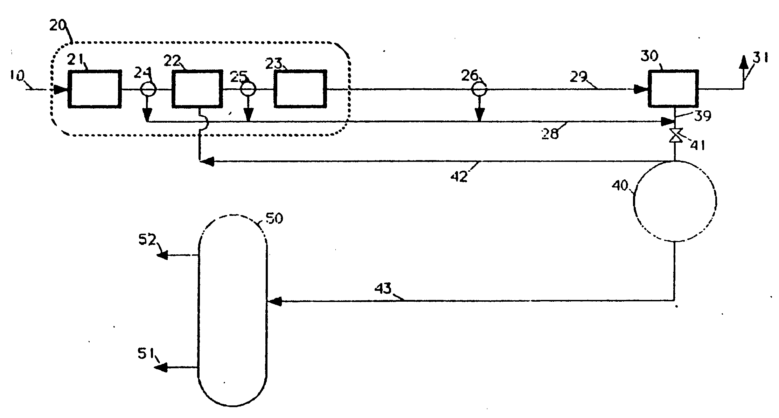

For a better understanding of this invention, reference is made to the detailed description which follows. together with the accompanying figure and subjoined claims.

## Brief Description of the Figure

The figure is a schematic diagram of the process and apparatus of the present invention as used or adapted for the separation of one or more selected $C_3$-$C_5$ olefinic products from light gases mixed therewith.

## Detailed Description of the Invention

As illustrated in the figure, in the process and apparatus of the present invention, the feed stream is a gas mixture typically including a desired $C_3$-$C_5$ olefinic product and lower boiling point compounds, such as C2 and C1 hydrocarbons, carbon oxides, hydrogen and nitrogen (produced for example in an upstream dehydrogenation process such as Air Products' Catofin or Catadiene processes; the feed stream comprises the cooled reactor output from such processes). In the present process, that mixed gas feed stream 10 is treated for separation of the $C_3$-$C_5$ olefinic product by pressurizing it in a compression train 20 to 100-500 psig, at 50-150°F.

Compression train 20 typically include 3 to 5 substages 21, 22 and 23 successively pressuring feed stream 10 to higher pressures. "Knockout" or "flash" drums 24, 25, 26 (including no pressure reduction such as to constitute true flash evaporation, however) are disposed on the outlet lines at one or more substages of the compression train so as to remove entrained liquid from pressurized vapor stream 29. The entrained liquid is collected as entrained liquid stream 28. While such "knockout" drums are shown on the outlet end of each of compression substages 21, 22 and 23, one or more of these knockout drums may not be necessary.

The pressurized vapor product of compression train 20 forms a vapor stream 29 which is cooled in a cryogenic or cooling stage 30 sufficiently to form a liquid condensate stream 39 having a light ends content of 1 to 30% (by weight), preferably 5 to 15%. This liquid, along with entrained liquid stream 28, at 50-150°F, is passed adiabatically to a flash evaporation stage 40 at a temperature through a pressure reducer, such as expansion valve 41. The remaining condensate stream 43 from flash evaporation stage 40, is fed to distillation stage 50 where it is separated into a light gas stream 52, which may optionally be recycled back to compression train 20, and a product olefinic stream 51. Product stream 51 includes a high concentration of the desired olefinic product, dependent on various intermediate parameters in the process.

Cryogenic cooling stage 30 may consist of any one or more, or a combination of, conventional cooling means. For example, all or part of pressurized vapor stream 29 may be adiabatically expanded as a means of cooling vapor stream 29 and condensing it to form liquid feed stream 39. Alternatively, a fraction of vapor stream 29 may be separated. compressed and then adiabatically cooled in combination with or subsequently to be combined with the remaining fraction of vapor stream 29 in order to effect the necessary cooling. Still another alternative is to cool vapor stream 29 by means of heat exchange with a refrigerant. In all cases, some light gases will be separated in the cooling sequence and this light gas stream 31 which is typically high in C1 and C2 hydrocarbons, as well as hydrogen, may be used as plant fuel gas.

The vapor fraction 42 of condensate 39 from flash evapcration stage 40 is recycled to compression stage 20. As illustrated, this recycle is shown to the second substage 22 of compression stage 20 but the precise entry of the recycle stream may be varied according to specific process design.

Distillation stage 50 typically comprises a multi-tray distillation column operated at a temperature at or slightly below that of the flash evaporation stage 40 varying from about 450 psig (where the olefinic product to be separated is propylene) to about 50 psig where the desired olefinic product is isoprene. Preferably, pressures of 50-150 psig are used when recovering isoprene, of 100-300 psig when recovering $C_4$ olefins, and about 400 psig when recovering propylene.

The vapor products of distillation stage 50, typically comprising a high concentration of hydrogen and C1 and C2 hydrocarbons. may also be used as plant fuel or may be recycled to the compression stage along with the vapor product stream 42 of flash evaporation stage 40. If the process is operated for the recovery of butadiene, the distillation stage 50 would be at low pressure, on the order of 100 psig; the vapor product stream 52 of distillation stage 50 will then be somewhat higher in product hydrocarbons. Therefore, in this embodiment of the process, it is preferred to recycle vapor stream 52 to the compression stage 20. Alternatively, if the desired product stream is primarily isobutylene, for example, with distillation stage 50 at a much higher pressure, on the order of 300 psig, the overhead vapor stream 52 of distillation stage 50 will be relatively low in the desired olefinic product and therefore will more likely be used as fuel gas, rather than recycled.

In the absence of flash evaporation stage 40, the efficiency of recovery of olefinic product in product stream 51 of distillation stage 50 (as a percentage of available product in feed stream 10) will either be greatly reduced or the size of distillation stage 50 must be commensurately greater in order to produce comparable product recovery efficiency.

While the intermediate stream pressure and temperature parameters in the process may be varied in order to adapt the process and apparatus to most efficient recovery of the desired olefinic product from a given feed stream, a determination and selection of these process parameters may easily be made by those skilled in the art depending on the specific feed stream at hand and the desired product. In order to assist in this determination and selection and to illustrate typical process parameters for specific processes, the following are computer-derived examples of the process of the present invention:

## Example I

An isobutane-rich paraffinic stream dehydrogenated in a conventional manner, such as a commercially operated Catofin process, produces a dehydrogenation product vapor stream rich in isobutylene. From this, a final product stream, comprised primarily of isobutylene, is to be separated in a process and apparatus, in accordance with the present invention. This apparatus includes a four-stage compression train and a cryogenic unit in which the pressurized vapor stream is cooled entirely by expansion cooling, i.e. permitting the stream to expand to a somewhat lower pressure with no energy input The process parameters and feed, final product and intermediate stream compositions are shown in TABLE I below, wherein streams are identified with the reference numerals of the Figure.

From the foregoing it will be noted that of the 39,011 kilograms per hour isobutylene in the feed stream to the separation process and apparatus of this invention, it is calculated that 38,926 kilograms per hour will be recovered. This indicates a recovery efficiency of about 99.8%. Such product recovery efficiency has heretofore not been obtainable except in more capital intensive stabilizer/absorber separation systems.

T A B L E   I

| STREAMS | 10 | 28 | 29 | 31 | 39 | 42 | 49 | 51 | 52 |
|---|---|---|---|---|---|---|---|---|---|
| Temperature (°C) | 40 | 40 | 15 | 35 | 33 | | | | |
| Pressure (Kg/cm²ABS) | 31.3 | 31.3 | 29.5 | 10.5 | 10.5 | | | | |
| Rate (Kg/Hr) | 83142 | 63509 | 21737 | 7408 | 14329 | 2106 | 75734 | 71753 | 3981 |
| Composition (as flow rate Kg/Hr) | | | | | | | | | |
| $H_2$ | 1616 | 46 | 1616 | 1606 | 10 | 46 | 10 | — | 10 |
| $C_1$ | 700 | 110 | 649 | 515 | 34 | 59 | 85 | — | 10 |
| Ethylene | 109 | 39 | 80 | 67 | 13 | 10 | 42 | — | 42 |
| Ethane | 133 | 56 | 88 | 67 | 21 | 11 | 99 | — | 99 |
| Propylene | 2291 | 1496 | 915 | 377 | 538 | 120 | 1914 | 105 | 1809 |
| Propane | 2114 | 1445 | 769 | 251 | 518 | 100 | 1863 | 251 | 1612 |
| Isobutane | 28796 | 23749 | 5685 | 59 | 5626 | 665 | 28710 | 28276 | 34 |
| Isobutylene ▪ | 39011 | 32867 | 6940 | 50 | 6890 | 796 | 38961 | 38926 | 35 |
| n-Butylene | 1030 | 874 | 176 | 176 | 20 | | 1030 | 1030 | |
| Butadiene | 660 | 562 | 111 | 111 | 13 | | 660 | 660 | |
| n-Butane | 1720 | 1484 | 266 | 266 | 30 | | 1720 | 1720 | |
| $C_{5}+$ | 385 | 383 | 2 | 2 | | | 385 | 385 | |
| $CO_2$ | 588 | 198 | 444 | 372 | 72 | 54 | 216 | — | 216 |
| CO | 223 | 14 | 221 | 217 | 4 | 12 | 6 | — | 6 |
| $N_2$ | 3793 | 186 | 3775 | 3727 | 48 | 168 | 99 | — | 99 |

▪ Desired Product

$$\% \text{ Recovery Available Product} = \frac{\text{in Stream 51}}{\text{Stream 10}} \times 100 = 99.8\%$$

Example II

This example is based on a system as described in Example I, with a feed stream rich in a desired final product comprised of butadiene.

The process parameters and stream compositions are similarly set forth, as for Example I, in Table II below.

The efficiency of the process is indicated by the 99.7% recovery of butadiene in final product stream 51, based on available butadiene in feed stream 10.

TABLE II

| STREAMS | 10 | 28 | 29 | 31 | 39 | 42 | 43 | 51 | 52 |
|---|---|---|---|---|---|---|---|---|---|
| Composition (as flow rate Kg/Hr) | | | | | | | | | |
| $H_2$ | 2082 | 45 | 2093 | 2082 | 11 | 54 | 2 | | |
| $C_1$ | 1350 | 184 | 1411 | 1350 | 61 | 199 | 46 | | 46 |
| Ethylene | 1284 | 596 | 1508 | 1284 | 224 | 465 | 355 | | 355 |
| Ethane | 1283 | 883 | 1623 | 1283 | 340 | 588 | 635 | | 635 |
| Propylene | 2631 | 8644 | 5452 | 2576 | 2876 | 2725 | 8795 | 55 | 8740 |
| Propane | 356 | 1518 | 723 | 261 | 462 | 402 | 1578 | 95 | 1483 |
| Isobutane | 1674 | 1696 | 327 | 17 | 310 | 191 | 1815 | 1657 | 158 |
| Isobutylene | 840 | 837 | 133 | 4 | 129 | 81 | 885 | 836 | 49 |
| $C_4=1$ | 13909 | 13781 | 2042 | 56 | 1986 | 1253 | 14514 | 13853 | 661 |
| Butadiene | 15949 | 15799 | 2249 | 48 | 2201 | 1399 | 16601 | 15901 | 700 |
| n-Butane | 56490 | 55682 | 6734 | 56 | 6678 | 4371 | 57989 | 56434 | 1555 |
| $C_4=2T$ | 16438 | 16167 | 1700 | 17 | 1683 | 1111 | 16739 | 16421 | 318 |
| $C_4=2C$ | 11801 | 11637 | 1332 | - | 1332 | 873 | 12096 | 11801 | 295 |
| $C_{5+}$ | 2709 | 2710 | 9 | - | 9 | 10 | 2709 | 2709 | - |
| $CO_2$ | 1607 | 667 | 1843 | 1607 | 236 | 527 | 376 | | 376 |
| CO | 370 | 19 | 378 | 370 | 8 | 25 | 2 | | 2 |
| $N_2$ | 5781 | 220 | 5893 | 5781 | 112 | 314 | 18 | | 18 |
| Rate (Kg/Hr) | 136554 | 131085 | 35450 | 16792 | 18658 | 14580 | 135185 | 119762 | 15393 |
| Temperature (°C) | | 40 | 40 | | 15 | 21 | ~21 | | |
| Pressure (Kg/cm²Abs.) | | 30.2 | 30.2 | | 29.5 | 4.2 | 4.2 | | |

99.7%

% Recovery
$$\frac{\text{Available Product in Stream 51}}{\text{Stream 10}} \times 100$$

Statement of Industrial Utility

This invention is expected to be useful in the cost effective separation of selected $C_3$-$C_5$ olefinic products from streams thereof including lower boiling point compounds. Such streams are typically produced in commercial paraffin dehydrogenation processes, such as the Catofin and Catadiene processes of Air Products and Chemicals Company.

6

**Claims**

1. A method for separating C₃-C₅ olefinic product from a gas stream comprised of said product and lower boiling point gases, said method comprising:

(a) compressing said stream to 100-500 psig at 50-150°F;

(b) condensing the compressed vapor product stream of step (a) by cooling said stream sufficiently to produce a condensate having 1 to 30% of said lower boiling point gases and removing the non-condensed vapor fraction thereof;

(c) flash evaporating the condensed stream product of step (b) at a pressure of 0-200 psig to remove at least some of said lower boiling point gases;

(d) distilling from the liquid stream product of step (c), substantially all of the lower boiling point gases remaining therein following said flash evaporation; and

(e) recovering from said distilling step (d) a liquid stream rich in said C₃-C₅ olefinic product.

2. The method of Claim 1 wherein said flash evaporation step (c) is conducted adiabatically.

3. The method of Claim 1 wherein said flash evaporation step (c) is conducted at a pressure of 50150 psig.

4. The method of Claim 1 wherein said condensation step (b) includes cooling said stream sufficiently to produce a condensate having 5 to 15% of said lower boiling point gases.

5. A method, as recited in Claim 1, wherein steps (a-e) comprise:

(a) compressing said stream to 100-500 psig at 50-150°F;

(b) condensing the compressed vapor product stream of step (a) by cooling said stream sufficiently to produce a condensate having 1 to 30% of said lower boiling point gases and removing the non-condensed vapor fraction thereof;

(c) flash evaporating said condensed stream at 0-200 psig to yield liquid and gaseous product streams;

(d) distilling said liquid product stream of step (c) at a pressure of from 100-300 psig; and

(e) recovering from said distilling step a liquid stream rich in isobutylene.

6. A method, as recited in Claim 1, wherein steps (a-e) comprise:

(a) step (a) comprises compressing said stream to 100-500 psig at 50-150°F;

(b) condensing the compressed vapor product stream of step (a) by cooling said stream sufficiently to produce a condensate having 1 to 30% of said lower boiling point gases and removing the non-condensed vapor fraction thereof;

(c) flash evaporating said condensed stream at 0-200 psig to yield liquid and gaseous product streams;

(d) distilling said liquid product stream of step (c) at a pressure of about 100 psig; and

(e) recovering from said distilling step a liquid stream rich in butadiene.

7. A method. as recited in Claim 1, wherein steps (a-e) comprise:

(a) compressing said stream to 100-500 psig at 50-150°F;

(b) condensing the compressed vapor product stream of step (a) by cooling said stream sufficiently to produce a condensate having 1 to 30% of said lower boiling point gases and removing the non-condensed vapor fraction thereof;

(c) flash evaporating said condensed stream at 0-200 psig to yield liquid and gaseous product streams;

(d) distilling said liquid product stream of step (c) at a pressure of 300-400 psig; and

(e) recovering from said distilling step a liquid stream rich in propylene.

8. A method, as recited in Claim 1, wherein steps (a-e) comprise:

(a) compressing said stream to 100-500 psig at 50-150°F;

(b) condensing the compressed vapor product stream of step (a) by cooling said stream sufficiently to produce a condensate having 1 to 30% of said lower boiling point gases and removing the non-condensed vapor fraction thereof;

(c) flash evaporating said condensed stream at 0-200 psig to yield liquid and gaseous product streams;

(d) distilling said liquid product stream of step (c) at a pressure of from 50-150 psig; and

7

(e) recovering from said distilling step a liquid stream rich in isopentadiene (isoprene).

9. A method as recited in Claim 1, wherein said distillation step (d) is conducted at a pressure the same as or slightly higher than said pressure of said flash evaporation step (c).

10. A method, as recited in Claim 1, wherein entrained liquid is removed from said gas stream at at least one point prior to said cooling step (b) and said removed entrained liquid is flash evaporated in said flash evaporation step (c) along with said liquid stream product of step (b).

11. A method, as recited in Claim 1, wherein the vapor stream from said flash evaporation step (c) is recycled to said compression step (a).

12. A method, as recited in Claim 1 wherein the distilled vapor stream from said distillation step (d) is recycled to said compression step (a).

13. A method, as recited in Claim 10 wherein the distilled vapor stream from said distillation step (d) is recycled to said compression step (a).

14. A method as recited in Claim 1 wherein the vapor stream from said flash evaporation step (c) is recycled to said compression step (a) and the distilled vapor stream from said distillation step (d) is recycled to said compression step (a).

15. A method, as recited in Claim 1 wherein said cooling step (b) comprises permitting at least a portion of said compressed gas stream from step (a) to expand adiabatically.

16. A method, as recited in Claim 1 wherein said cooling step (b) comprises compressing a fraction of said compressed gas stream from step (a) and then permitting said fraction to expand adiabatically in the presence of the remainder of said compressed gas stream product from step (a).

17. A method, as recited in Claim 1, wherein said cooling step (b) comprised heat exchanging said compressed gas stream with an external refrigerant.

18. Apparatus for separating $C_3$-$C_5$ olefinic product from a gas stream comprised of said product and lower boiling point gases, said apparatus comprising:

(a) means for compressing said stream to 100-500 psig;

(b) means for condensing the compressed vapor product stream of means (a) by cooling said stream sufficiently to produce a condensate having 1 to 30% of said lower boiling point gases and removing the non-condensed vapor fraction thereof;

(c) means for flash evaporating the condensed stream product of step (b) at a pressure of 0-200 psig to remove at least some of said lower boiling point gases;

(d) means for distilling from the liquid stream product of step (c) substantially all of the lower boiling point gases remaining therein following said flash evaporation; and

(e) means for recovering from said distilling step (d) a liquid stream rich in said $C_3$-$C_5$ olefinic product.